# EUROPEAN PATENT APPLICATION

(11) **EP 1 531 156 A1**
(43) Date of publication of application: **18.05.2005**
(21) Application number: 04026771.8
(22) Date of filing: 11.11.2004
(51) Int. Cl.: C07F 7/18, A61K 31/695

(54) **Method for synthesis of silylated ascorbic acid derivatives**

(30) Priority: 17.11.2003 US 520794 P; 25.10.2004 US 972465
(71) Applicant: CLARIANT INTERNATIONAL LTD., 4132 Muttenz (CH)
(72) Inventor: Terry, Leonhard W., Gainsvill, Florida 32653 (US); Legrow, Gary E., Newberry, Florida 32669 (US); Littau, Cheryl Ann, Dr., Charlotte, NC 28211 (US); Landtiser, Richard, Gainesville, Florida 32607 (US)
(74) Representative: Paczkowski, Marcus, Dr.

(57) **Abstract**

A compound of Formula (I), as defined in the specification, and a method for its synthesis. The method includes providing an inert environment, minimizing exposure of the reaction environment to visible light and reacting ascorbic acid with solvent and a compound of the Formula (II), as defined in the specification. The method of the present invention produces increased yields and a compound having increased stability and the same or greater bioactivity than ascorbic acid. The compound formed by the method of the present invention is capable for use in anhydrous cosmetic, dermatological and pharmaceutical compositions as an active agent for combating aging of the skin and improving its aesthetic appearance.

## Description

The present invention relates generally to methods for the production of silylated compounds, and in particular, to a method for the synthesis of silylated ascorbic acid derivatives.

Ascorbic acid (Vitamin C) has long been recognized in the cosmetic, personal care and pharmaceutical industries as a useful compound for combating various skin conditions and for maintaining youthful, healthy looking skin. Ascorbic acid helps to decrease the presence of wrinkles as it contributes to the production of collagen.
The compound is also known to whiten the skin by reducing production of melanin, and to reduce the harmful effects of sun exposure. In consequence, the cosmetics and pharmaceutical industries have long sought to utilize ascorbic acid in a wide range of products to capture its beneficial properties. Despite its attributes, however, ascorbic acid suffers from a lack of stability in aqueous formulations. Moreover, ascorbic acid is highly insoluble in nonaqueous formulations. These disadvantages severely hamper the use of ascorbic acid in cosmetic, personal care and pharmaceutical compositions.

Many attempts to incorporate ascorbic acid into nonaqueous products, for example, esters, fatty acids and fatty alcohols, produce low levels of incorporation.
US 6,146,664 discloses that suspensions of solid ascorbic acid in nonaqueous silicone carriers results in systems that stabilize the ascorbic acid, while still maintaining high bioavailability/high efficacy. While maintenance of efficacy was achieved by US 6,146,664, the method advanced therein results in the potential for physical instability (settling) of the solid ascorbic acid, less than optimal aesthetic characteristics given by the suspended solid particles, and the potential for uneven coverage due to insolubility. The potential for locally high concentrations in cosmetic, dermatological and pharmaceutical compositions increases the probability for dermal irritation, resulting in an undesirable product. Furthermore, concentration gradients invariably produce regions with low or no active ingredient, thereby directly impacting the composition's efficacy.

In response to the inability to directly incorporate ascorbic acid into formulations, the industries have turned their attention to synthesizing derivatives of ascorbic acid and incorporating such derivatives into the cosmetic and dermatological compositions, hoping to achieve the beneficial bioactivity, while avoiding the problems associated with ascorbic acid. These attempts have been generally unsatisfactory. The majority of derivatives successfully synthesized and isolated have not exhibited a level of bioactivity which justifies their inclusion. In other attempts, the industry has been incapable of synthesizing and isolating derivatives thought to manifest the same or superior bioactivity as compared to ascorbic acid.

As a result, there exists a need for a method for synthesizing a derivative of ascorbic acid that would be bioactive, more stable to air oxidation, and soluble in non-aqueous systems.

Accordingly, in one preferred aspect, the present invention is directed to a method for synthesizing a silylated ascorbic acid derivative of the Formula (I) and the product so formed. 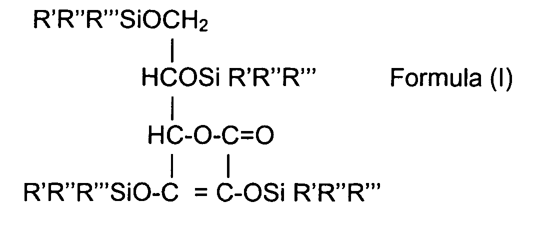 wherein R', R" and R"' are, independently unbranched or branched C₁₋₈ alkyl or CH₂=CH-.
The method of synthesis includes providing an inert reaction environment prior to reaction and reacting ascorbic acid in a compatibilizing solvent with a compound of Formula (II)

R'R"R"'Si-NH-Si R'R"R"' Formula (II)

Preferably, the reaction takes place in an environment free of visible and ultraviolet radiation. Maintaining a reaction environment free of visible and UV radiation and in an inert atmosphere permits the conversion to, and isolation of, compounds of the Formula (I) in an actual yield generally greater than or equal to 95% of theoretical yield. This yield was heretofore unattainable and permits the use of the compound in cosmetic and dermatological applications.

According to another preferred aspect, the invention is directed to a composition comprising at least one compound of Formula (I). As these ascorbic acid derivatives are soluble in non-aqueous solutions, employing these derivatives in anhydrous cosmetic, dermatological and pharmaceutical formulations yields the same or greater functional benefits seen with ascorbic acid without the insolubility problems encountered therewith.

These and other objects, advantages and features of this invention will become apparent upon review of the following specification.

### Detailed Description Of The Preferred Embodiments

The present invention is, in one aspect, directed to compounds of Formula (I): 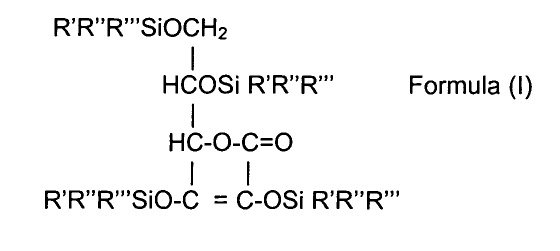 wherein R', R" and R"' are independently unbranched or branched C₁₋₈ alkyl or CH₂=CH-. Preferably, R', R" and R"' are a C₁₋₂ alkyl and are most preferably CH₃.

In another aspect, the invention is directed to a process for the synthesis of compounds of Formula (I). The process includes providing an inert reaction environment. Once an inert atmosphere is achieved, ascorbic acid is reacted in a compatibilizing solvent with a compound of Formula (II)

R'R"R"'Si-NH-Si R'R"R"' Formula (II)

wherein R', R" and R"' are as defined above.
The reaction is conducted in conditions that minimize the introduction to, or exposure of, the reaction environment to visible and preferably, ultraviolet light. Preferably, the reaction is conducted in the absence of both visible and ultraviolet light. In a preferred embodiment, the reaction is conducted in the presence of an acid catalyst. Preferably, the acid catalyst is sulfuric acid or trifluoromethanesulfonic acid, otherwise known as triflic acid. Most preferably the acid catalyst is trifluoromethanesulfonic acid.

As used herein, the term "compatabilizing solvent" means any solvent capable of dissolving ascorbic acid, which is inert, stable and can be separated from the final compound. Preferably, the compatibilizing solvent is also recyclable. Non-limiting examples of such solvents include, but are not limited to, polar aprotic organic solvents such as cycloaliphatic ether, for example, tetrahydrofuran; an acyclic aliphatic ether, for example dibutyl ether, and a dialkylether of polyalkylene oxide, for example, ethylene glycol dimethyl ether. Preferably, the solvent is ethylene glycol dimethyl ether or tetrahydrofuran, most preferably, tetrahydrofuran.
As used herein "inert atmosphere" means the absence of moisture (less than 200 ppm H₂O and oxygen (less than 500 ppm).
The reaction between the ascorbic acid, compatibilizing solvent and the compound of Formula (II) is maintained at a temperature of between approximately 20°C and 150°C, preferably 30°C to 90°C and most preferably 40°C to 80°C for a time period necessary to dissolve the ascorbic acid. Dissolution of the ascorbic acid is identifiable by the presence of a substantially clear, translucent pale yellow colored reaction solution.
Utilizing the method of the present invention permits the production of the ascorbic acid derivative, Formula (I), in high yields, i.e. greater than or equal to 95% theoretical yield. The compounds of Formula (I), furthermore, exhibit the same beneficial characteristics as ascorbic acid when used in anhydrous cosmetic, dermatological or pharmaceutical compositions and do not present the problem of settling attendant the use of ascorbic acid and exhibit greater stability.

According to another aspect, the present invention is embodied in anhydrous cosmetic, dermatological or pharmaceutical composition including an effective amount of at least one ascorbic acid derivative compound of Formula (I) with a suitable vehicle or carrier. As used herein, the term "anhydrous" means containing water in an amount of 1% or less. The compounds of Formula (I) may be added to any topical, anhydrous cosmetic, dermatological or pharmaceutical composition as an effective agent to impart upon such compositions the combat aging of the skin in all its forms, such as, for example, treating, preventing or reducing skin dispigmentation, oxidative damage, photoaging, wrinkles and fine lines. The compositions of the present invention may take the form of, for example, a solution, a serum, an anhydrous gel, stick, lipstick, lotion, or ointment.

The amount of the compound of Formula (I) present in the composition administered is application specific, depending upon the desired effect, and thus, can vary to a great extent. In the majority of applications, the amount of the compound of Formula (I) administered ranges from 0.1 to 50 %, and preferably, from 1 to 25% by weight based on the total weight of the composition. However, it will be realized by those with ordinary skill in the art that any effective amount of a compound of Formula I may be used to arrive at the desired effect without departing from the spirit and scope of the present invention.

The compositions may be based on any anhydrous system normally employed in the cosmetic, dermatological and/or pharmaceutical field. Preferably, the carrier is oil based. Examples of oils suitable for use in the present invention include, but are not limited to mineral oils, for example, liquid paraffin; petrolatum; vegetable oils, for example, liquid fraction of shea butter or sunflower oil; animal oils, for example, perhydrosqualene; synthetic oils, for example, polydecene; silicone oils for example, cyclomethicone or dimethicone; organosilicone oils and waxes, for example, SilCare® Silicone 41M15 (Caprylyl Methicone), SilCare® Silicone 31 M series (Caprylyl Trimethicone), SilCare® Silicone 15M series (Phenyl Trimethicone) or SilCare® Silicone 41M90 (C₃₀₊ alkyldimethicone); and fluorinated oils, for perfluoro polyethers). Fatty alcohols, fatty acids, for example stearic acid and waxes such as paraffin, carnauba, beeswax may also be used as fatty substances.

As is known by those with ordinary skill in the art, the compositions may also contain adjuvants which are customary in the cosmetics, pharmaceutical or dermatological field. Non-limiting examples of such adjuvants include lipophilic gelling agents, hydrophilic or lipophilic active agents, preservatives, antioxidants, solvents, perfumes, fillers, sunscreen agents, bactericides, odor absorbers and colorants. The concentrations of these different adjuvants are those traditionally used in the cosmetic, pharmaceutical or dermatological field, and are, for example, from 0.01 % to 30% of the total weight of the composition. The adjuvants, depending on their nature, may be introduced into the fatty phase and/or into lipid spherules.

The composition can also combine two or more compounds of Formula (I), alone or with other active agents. Non-limiting examples of such other active agents include retinol derivatives (especially retinoxytrimethylsilane, available commercially from Clariant as SilCare® Silicone 1 M75), trimethylsilylderivatives of alphahydroxyacids (commercially available from Clariant as SilCare® Silicone 180M10 and SilCare® Silicone 180M20) or trimethylsilylderivatives of betahydroxyacids (commercially available from Clariant as SilCare® Silicone 180M30), tocopherol and derivatives thereof, ceramides, and essential oils.

The composition may also have one or more of the following ingredients or adjuvants: anesthetics, antibacterials, antifungals, steroidal anti-inflammatory agents, antidandruff agents, antiacne agents, chelating agents, antioxidants, stabilizers, colorants, emollients, fragrances, humectants, lubricants, preservatives, skin penetration enhancers, thickeners, viscosity modifiers, vitamins, moisturizers or any mixtures thereof.

The compositions of the present invention are topically applied to individuals in a manner customary with other cosmetic, dermatological or pharmaceutical compositions. The amount applied, frequency of application and duration of treatment is variable so as to enable achievement of the desired appearance of the skin and is within the purview of an artisan with ordinary skill.

The following are illustrative, non-limiting examples of the present invention.

### Examples

### Example 1: Synthesis of tetrakis(trimethylsilyl)ascorbate

All of the synthetic runs that lead to a high conversion of Ascorbic Acid (Vitamin C) to Tetrakis(trimethylsilyl)ascorbate involved the use of hexamethyldisilazane. Table I, below, shows the variations tried that ultimately led to a high quality, high conversion, product.

**Table I**

| Rxn # | Solvent | Temperature | Over Gas | Light Exposure | Catalyst | Conversion | Product Chars |
|---|---|---|---|---|---|---|---|
| 1 | THF | Reflux | Air | Fluorescent | None | None | |
| 2 | Neat | Reflux | Air | Fluorescent | None | None | |
| 3 | DGDME | Reflux | Air | Fluorescent | None | Low | Deep Red |
| 4 | Methanol | Reflux | Air | Fluorescent | None | None | |
| 5 | DGDME | Reflux | Nitrogen | Fluorescent | None | Medium | Pale Orange |
| 6 | DGDME | Reflux | Nitrogen | Dark | None | High^{+*} | Pale Yellow |
| 7 | THF | Reflux | Nitrogen | Dark | Triflic Acid | Quantitative | Pale Yellow |

| | | | | | | | |
|---|---|---|---|---|---|---|---|
| *NMR of compound has four separate Me₃Si peaks (two pairs) with identical integrations. *GC of the product is a single sharp peak. | | | | | | | |
| ⁺ "High," as used herein, means that an actual yield of 95% or greater of the theoretical yield was achieved. | | | | | | | |

Following is the methodology of run 6, which was repeated several times.

A three neck 500 ml round bottom flask was set up with a mechanical stirrer, heating mantle, thermocouple linked to a temperature controller, reflux condenser, addition funnel and a fritted glass tube to allow entry of dry nitrogen gas subsurface to the liquid contents of the flask. To the flask was added 35.3 g (0.20 mole) of ascorbic acid and 200 g of Ethylene Glycol Dimethyl Ether. Nitrogen gas was allowed to enter the flask through the glass frit subsurface to the suspension in the flask for a period of 18 hours in order to purge the vessel of all air. The flask was then covered in aluminum foil to exclude light, and heated to 50°C with stirring. 70.9 g (0.44 mole) of hexamethyldisilazane was added to the flask and the temperature was maintained at 50°C for 8 hours with stirring. During this time, the contents of the flask were transformed from a white slurry to a pale peach colored solution. A GC analysis of the solution showed three peaks, identified as EGDME, hexamethyldisilazane and the product. After removing the N₂ subsurface glass frit, a full vacuum was applied to the flask, maintaining the temperature at 50°C to remove volatiles. Approximately 200 g of volatiles were removed from the flask which were identified as ~97% EGDME and ~3% hexamethyldisilazane. A GC analysis of the product remaining in the flask showed a single peak. The product left in the flask weighed 88 g. (95% yield relative to theoretical yield). A sample of the product in the flask was analyzed by NMR and shown to have four separate trimethylsilyl peaks (actually two pairs of peaks), the pairs centered at 0 ppm and 0.2 ppm. The approximate normalized relative integrations of the four peaks were 1:1:1:1.

### Example 2: Synthesis of tetrakis(trimethylsilyl)ascorbate

Following is the methodology of run 7, which was repeated several times.

A three neck 500 ml round bottom flask was set up with a magnetic stir bar, heating mantle, thermocouple linked to a temperature controller, reflux condenser, addition funnel and a fritted glass tube to allow entry of dry nitrogen gas subsurface to the liquid contents of the flask. To the flask was added 75.8 g (1.5 mole) of ascorbic acid, 250 g of THF and 100 µL of triflic acid. The flask was then covered in aluminum foil to exclude light, and heated to 66°C with stirring, 305 g (3.3 mole) of hexamethyldisilazane was added to the flask and the temperature was maintained at 70°C for 6 hours with stirring. During this time, the contents of the flask were transformed from a white slurry to a pale yellow colored solution. A GC analysis of the solution showed three peaks, identified as THF, hexamethyldisilazane and the product. Finally, 2 g of Calcium Carbonate was added to the reaction flask and stirred for 2 hours. After filtration, the flask was set up to distill any low boiling solvents off. An additional 100 g of hexamethyldisiloxane was added and the reaction mixture heated to 70°C. Approximately 250 g of volatiles were removed from the flask, which were identified as ~99% THF. The flask was then heated to 100°C under a vacuum of 100 mmHg to remove any hexamethyldisiloxane and hexamethyldisilazane from the reaction flask. Approximately 90 g was collected during this step which was identified as 97% hexamethyldisiloxane and ~3% hexamethyldisilazane. Finally, a nitrogen sparge was re-inserted and the flask was put under full vacuum for 30 minutes. A GC analysis of the product remaining in the flask showed a single peak. The product left in the flask weighed 198 g. (99% yield relative to theoretical yield). A sample of the product in the flask was analyzed by GC and identified as the same compound identified in Example 1.

### Example 3: Examples of the Composition

### Example 3a:

An anhydrous treatment gel composition of the present invention was prepared comprising the following ingredients: about 20 wt. % tetrakis (trimethylsilyl) ascorbate, caprylic/capric triglycerides, SilCare® SiliCone 41M15 (Caprylyl Methicone), SilCare® Silicone 41M80 (C₂₄₋₂₈ Alkyl dimethicone), microcrystalline wax, SilCare® Silicone 31M50 (Caprylyl Trimethicone), tocopherol acetate, caprylic/capric/stearic triglyceride, and fragrance.

### Example 3b:

An anhydrous serum composition of the present invention was prepared comprising the following ingredients: about 10 wt. % tetrakis (trimethylsilyl) ascorbate, caprylic/capric triglycerides, SilCare® SiliCone 41M15 (Caprylyl Methicone), SilCare® Silicone 31M50 (Caprylyl Trimethicone), tocopherol acetate, and fragrance.

### Example 3c:

An anhydrous treatment gel composition of the present invention was prepared comprising the following ingredients: about 10 wt. % tetrakis (trimethylsilyl) ascorbate, about 10 wt% SilCare Silicone 180M20 (trimethylsilyl Trimethylsiloxy Glycolate), about 10 wt. % 180M 30 (Trimethylsilyl Trimethylsiloxy Salicylate), caprylic/capric triglycerides, SilCare® SiliCone 41M15 (Caprylyl Methicone), SilCare® Silicone 41M90 (C₃₀₊ Alkyl dimethicone), microcrystalline wax, SilCare® Silicone 31M50 (Caprylyl Trimethicone), tocopherol acetate, caprylic/capric/stearic triglyceride, and fragrance.

### Example 3d:

An anhydrous serum composition of the present invention was prepared comprising the following ingredients: about 10 wt. % tetrakis (trimethylsilyl) ascorbate, about 10 wt% SilCare® Silicone 1M75 (retinoxytrimethylsilane), caprylic/capric triglycerides, SilCare® Silicone 41M15 (Caprylyl Methicone), SilCare® Silicone 31M50 (Caprylyl Trimethicone), tocopherol acetate, fragrance.

It will be readily understood by those persons skilled in the art that the present invention is susceptible of broad utility and application. Many embodiments and adaptations of the present invention other than those herein described, as well as many variations, modifications and equivalent arrangements, will be apparent from or reasonably suggested by the present invention and the foregoing description thereof, without departing from the substance or scope of the present invention. Accordingly, while the present invention has been described herein in detail in relation to its preferred embodiment, it is to be understood that this disclosure is only illustrative and exemplary of the present invention and is made merely for purposes of providing a full and enabling disclosure of the invention. The foregoing disclosure is not intended or to be construed to limit the present invention or otherwise to exclude any such other embodiments, adaptations, variations, modifications and equivalent arrangements.

## Claims

1. A compound of the Formula (I) 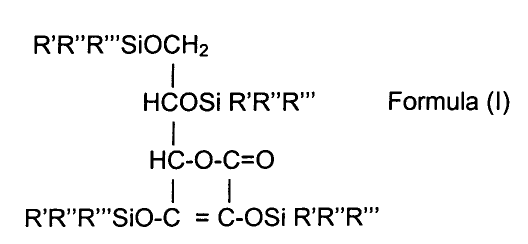 wherein R', R" and R"' are independently unbranched or branched C₁₋₈ alkyl or CH₂=CH-.

2. A compound as claimed in claim 1, wherein R', R" and R"' are a C₁₋₂ alkyl.

3. A compound as claimed in claim 2, wherein R', R" and R"' are CH₃.

4. A compound as claimed in claim 1, wherein R', R" and R"' are CH₂=CH-.

5. A cosmetic, dermatological or pharmaceutical composition comprising at least one compound as claimed in at least one of claims 1 to 4.

6. A composition as claimed in claim 5, wherein said at least one compound is from 0.1 % to 75% by weight of the total weight of the composition.

7. A composition as claimed in claim 6, wherein said at least one compound is from 0.5% to 50% by weight of the total weight of the composition.

8. A composition as claimed in claim 7, wherein said at least one compound is from 0.5% to 25% by weight of the total weight of the composition.

9. A composition as claimed in at least one of claims 5 to 8, wherein said composition is a solution, dispersion, serum, gel, stick, lipstick, ointment or lotion.

10. A composition as claimed in at least one of claims 5 to 9, further comprising at least one additive or adjuvant.

11. A composition as claimed in at least one of claims 5 to 10, further comprising at least one active agent.

12. A method for making a compound of the Formula (I) 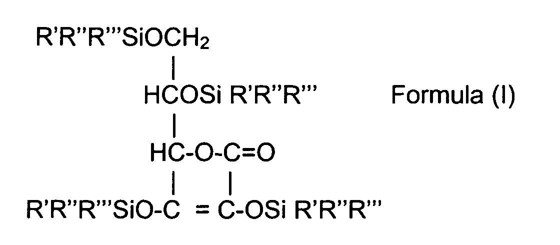 wherein R', R" and R"' are independently unbranched or branched C₁₋₈ alkyl or CH₂=CH- comprising the steps of:
providing an inert reaction environment;
reacting, in said inert reaction environment, ascorbic acid in a compatibilizing solvent with a compound of the Formula (II)
R'R"R"'Si-NH-Si R'R"R"' Formula (II)
wherein R', R" and R"' are as defined in Formula (I).

13. A method as claimed in claim 12, further comprising the step of minimizing exposure of said inert reaction environment to visible light.

14. A method as claimed in claim 13, wherein said minimizing step further comprises minimizing exposure of said reaction environment to ultraviolet light.

15. A method as claimed in claim 13 or 14, wherein said minimizing step further comprises absence of visible light in said inert reaction environment.

16. A method as claimed in claim 14 or 15, wherein said minimizing step further comprises absence of ultraviolet light.

17. A method as claimed in at least one of claims 12 to 16, wherein the reacting step occurs in the presence of an acid catalyst.

18. A method as claimed in claim 17, wherein the acid catalyst is selected from the group consisting of sulfuric acid and trifluoromethanesulfonic acid.

19. A method as claimed in claim 18, wherein the acid catalyst is trifluoromethanesulfonic acid.

20. A method as claimed in at least one of claims 12 to 19, wherein R', R" and R"' are C₁₋₂ alkyl.

21. A method as claimed in claim 20, wherein R', R" and R"' are CH₃.

22. A method as claimed in at least one of claims 12 to 19, wherein R', R" and R"' are CH₂=CH-.

23. A method for combating aging of skin comprising the step of administering to a patient a therapeutically effective amount of a composition as claimed in at least one of claims 5 to 11 for a period of time necessary to improve the condition of the skin.
